# EUROPEAN PATENT APPLICATION

(11) **EP 1 757 275 A1**
(43) Date of publication of application: **28.02.2007**
(21) Application number: 05734699.1
(22) Date of filing: 21.04.2005
(51) Int. Cl.: A61K 9/48, A61K 31/335, A61K 47/02, A61K 47/04, A61K 47/06, A61K 47/14, A61K 47/36, A61K 47/42, A61K 47/44, A61P 37/08

(54) **SEAMLESS CAPSULE CONTAINING WATER-SOLUBLE ACTIVE INGREDIENT**

(30) Priority: 21.04.2004 JP 2004125485
(71) Applicant: KYOWA HAKKO KOGYO CO., LTD., Chiyoda-ku, Tokyo 100-8185 (JP)
(72) Inventor: MURAI, Kouji c/o Kyowa Hakko Kogyo C., Ltd., Chiyoda-ku, Tokyo 100-8185 (JP); KATO, Yasuki, Shizuoka 410-1115 (JP)
(74) Representative: Harding, Charles Thomas
(86) International application number: PCT/JP2005/007624
(87) International publication number: WO 2005/102291

(57) **Abstract**

An object of the present invention is to provide a seamless capsule containing a water-soluble active substance in which the water-soluble active substance is protected from becoming unstable; and a process for producing the same. The seamless capsule comprises: content comprising a water-soluble active substance and an oily compound; a shell covering the content; and an intermediate layer, which is laying between the content and the shell and comprises an oily compound which does not dissolve or merge with the water-soluble active substance, is not dissolved in or merged with water, has a melting point of 10 to 65°C, and is not dissolved in or merged with the content at a temperature not higher than the melting point during the production process of the seamless capsule is provided. The process for producing the seamless capsule containing a water-soluble active substance using a device for producing the seamless capsule equipped with three or more layered nozzles with sequentially increasing diameters is provided.

## Description

### Technical Field

The present invention relates to a seamless capsule containing a water-soluble active substance and a process for producing the same.

### Background Art

In solid products containing an active substance for medical drugs or animal drugs, many active substances for medical drugs or animal drugs are soluble in water, and there are many reports that such an active substance is unstable when water are used in the production of solid products or water are contained in solid products. It is considered that unstableness is caused by a chemical reaction of the active substance with water or the active substance becoming sensitive to other component of preparation or an external factor due to being dissolved in water. Accordingly, in the production of solid products containing an active substance for medical drugs or animal drugs, particularly, a water-soluble active substance, various methods of protecting the active substance from being affected by water used in the production of solid products or water contained in solid products have been studied.

On the other hand, a seamless capsule is produced by, for example, a process described in JP-B-51-08875 or the like. The seamless capsule described in JP-B-51-08875 has a structure in which content, which is an oily compound, is encapsulated with a water-soluble shell, and is called a mono-nuclear single-layered seamless capsule. On the other hand, a seamless capsule which has a multi-layered structure composed of content, a shell covering the content and further an intermediated layer laying between the content and the shell, and is called a mono-nuclear two-layered seamless capsule is also known (see Patent document 1). In the mono-nuclear two-layered seamless capsule, the content is an oily compound containing a water-soluble or a water-insoluble solid dispersed therein, the intermediate layer is an emulsified liquid composed of oil and a water-soluble polymer such as gelatin, and the content and the intermediate layer are encapsulated with a water-soluble shell.

Further, the application of a seamless capsule having a multi-layered structure has been reported (see Patent documents 2 to 5), and for example, a seamless capsule having a structure in which content that is an aqueous solution or an aqueous suspension comprising a water-soluble substance and an intermediate layer composed of an oily compound are encapsulated with a water-soluble shell is known. By these techniques, seamless capsules containing content such as a drug, a flavor, an animal oil or a plant oil have been widely used in various applications.

Further, as a seamless capsule containing a substance which is insoluble in water among active substances for medical drugs or animal drugs, a seamless capsule containing a useful enteric bacterium as content (see Patent document 6) and a seamless capsule containing an oily compound which is easily oxidized as content (see Patent document 7) are known. In these seamless capsules, content which is an oily compound and an intermediate layer composed of an oily compound which is not flowable at room temperature are encapsulated with a water-soluble shell.
Patent Document 1: JP-B-53-39193
Patent Document 2: Japanese Patent No. 2806564
Patent Document 3: Japanese Patent No. 2784872
Patent Document 4: JP-A-10-313861
Patent Document 5: JP-A-2001-238611
Patent Document 6: JP-B-7-61948
Patent Document 7: JP-A-7-53356

### Disclosure of the Invention

### Problems to be Solved by the Invention

An object of the present invention is to provide a seamless capsule containing a water-soluble active substance in which the water-soluble active substance is protected from becoming unstable and a process for producing the same.

### Means for Solving the Problems

The present invention relates to the following (1) to (9):
(1) A seamless capsule comprising: content comprising a water-soluble active substance and an oily compound; a shell covering the content; and an intermediate layer, which is laying between the content and the shell and comprises an oily compound which does not dissolve or merge with the water-soluble active substance, is not dissolved in or merged with water, has a melting point of 10 to 65°C, and is not dissolved in or merged with the content at a temperature not higher than the melting point during the production process of the seamless capsule;
(2) The seamless capsule according to the above (1), wherein the intermediate layer is an intermediate layer comprising an oily compound having a melting point of 25 to 37°C;
(3) The seamless capsule according to the above (1) or (2), wherein the intermediate layer is an intermediate layer comprising one or more oil selected from hard fats, cacao oil, lard, hydrogenated oil, partially hydrogenated oil, glycerol esters of fatty acids, fatty acid acyl esters and paraffin;
(4) The seamless capsule according to the above (1) or (2), wherein the intermediate layer is an intermediate layer comprising a mixture of one or more oil selected from hard fats, cacao oil, lard, hydrogenated oil, partially hydrogenated oil, high-melting glycerol esters of fatty acids, high-melting fatty acid acyl esters and paraffin with one or more oil selected from plant oil, low-melting glycerol esters of fatty acids, low-melting fatty acid acyl esters and liquid paraffin;
(5) The seamless capsule according to any one of the above (1) to (4), wherein the shell is a shell comprising gelatin or agar ;
(6) The seamless capsule according to the above (5), wherein the shell is a shell comprising one or more substance(s) selected from titanium oxide, iron oxide, magnesium oxide, calcium oxide, zinc oxide, calcium carbonate, talc, silicon oxide, aluminosilicates, sodium copper chlorophyllin and aluminum lakes of tar dyes;
(7) The seamless capsule according to any one of the above (1) to (6), wherein the water-soluble active substance is olopatadine or a salt thereof;
(8) A process for producing a seamless capsule comprising a water-soluble active substance, which comprises the steps of: with the use of a device for producing the seamless capsule equipped with three or more layered nozzles with sequentially increasing diameters, forming a multi-layered droplet by continuously discharging to a cooling medium, a encapsulating solution from the outermost nozzle, an oily compound constituting an intermediate layer comprising the oily compound which does not dissolve or merge with a water-soluble active substance, is not dissolved in or merged with water, has a melting point of 10 to 65°C, and is not dissolved in or merged with content of the seamless capsule at a temperature not higher than the melting point during the production process of the seamless capsule from at least one nozzle other than the innermost nozzle among nozzles other than the outermost nozzle, and the content comprising the water-soluble active substance and an oily compound from at least one nozzle inside the nozzle discharging the oily compound constituting the intermediate layer, respectively; hardening or gelating the oily compound constituting the intermediate layer by cooling the multi-layered droplet to a temperature not higher than the melting point of the oily compound constituting the intermediate layer at the same time as or immediately after the formation of the droplet; and drying the resulting solid matter at a temperature not higher than the melting point of the oily compound constituting the intermediate layer;
(9) A seamless capsule containing a water-soluble active substance that can be produced by the process for producing a seamless capsule according to the above (8).

### Effect of the Invention

According to the present invention, a seamless capsule containing a water-soluble active substance in which the water-soluble active substance is protected from becoming unstable, and a process for producing the same can be provided.

### Best Mode for Carrying Out the Invention

A seamless capsule of the present invention comprises: content comprising a water-soluble active substance; a shell covering the content; and an intermediate layer laying between the content and the shell, and is characterized in that the content comprise the water-soluble active substance and an oily compound, and the intermediate layer comprises an oily compound which does not dissolve or merge with the water-soluble active substance, is not dissolved in or merged with water, has a melting point of 10 to 65°C, and is not dissolved in or merged with the content at a temperature not higher than the melting point.

The oily compound constituting the content of the seamless capsule of the present invention may be any an oily compound as long as it is conventionally used as content of a seamless capsule. Further, a water-soluble oily compound which has not been used as content of a conventional seamless capsule can also be used as the oily compound constituting the content of the seamless capsule of the present invention. The water-soluble active substance may be unstable against water in some cases, therefore, it is preferred that the content do not comprise water. Further, as the oily compound, those capable of suspending the water-soluble active substance. Further, an oily compound with low viscosity can be used by mixing it with an oily compound with high viscosity.

Examples of the oily compound constituting the content of the seamless capsule of the present invention comprise one oily compound or a mixture of two or more oily compounds selected from oil and fats such as medium-chain fatty acid triglycerides and soybean oil, alcohols such as glycerol and ethanol, surfactants derived from oil and fats such as sucrose esters of fatty acid and polyoxyethylene hydrogenated castor oil, synthetic surfactants such as polysorbate and polyethylene glycols, non-polar hydrocarbons such as liquid paraffin, normal paraffin having 6 or more carbon atoms and the like.

The water-soluble active substance to be contained in the seamless capsule of the present invention may be any active substance as long as it is slightly soluble in water. For example, it is preferably one with a solubility of 0.1 mg/mL or higher, that is, with solubility higher than that represented by "very slightly soluble" defined in the Japanese Pharmacopoeia. Further, in terms of the distribution of water and oil, it may be any active substance that is slightly distributed in water. For example, one with a distribution ratio of octanol to water of 1:0.01 or higher is preferred. More preferably, for example, the solubility in water is 10 mg/mL or higher and the distribution ratio of octanol to water is 1:10 or higher Further, a water-soluble active substance with an extremely low stability against water, for example, a peptide, an antibody, DNA or the like, or a water-soluble active substance which generates a specific change in property (such as change in color) when it comes into contact with water or the like can be used in the present invention without regard to the solubility in water and the distribution ratio of water to oil.

Further, the water-soluble active substance to be contained in the seamless capsule of the present invention is preferably one which is suspended in an oily compound to form content. In this case, even if a part of the active substance is dissolved in or merged with the oily compound, it does not matter as long as not all the active substance is dissolved in or merged with the oily compound.

As the form of the water-soluble active substance to be contained in the seamless capsule of the present invention, a solid, a powder, a crystal and the like are preferred. Further, in the case of a water-soluble active substance that is dissolved in or merged with an oily compound or a water-soluble active substance that is sensitive to generate an aggregate or the like, it is preferred to prepare in a form that is easily suspended in an oily compound constituting the content by including it in a clathrate compound such as cyclodextrin, encapsulating it in a liposome or a microcapsule or adsorbing it by an adsorptive substance such as silica gel or pregelatinized starch.

As the water-soluble active substance to be contained in the seamless capsule of the present invention, for example, a water-soluble active substance for a medical drug or an animal drug is preferred, and more specific examples thereof include one or more biologically active substance(s) and the like selected from the group consisting of central nervous system drugs, peripheral nervous system drugs, sensory organ drugs, cardiovascular drugs, respiratory drugs, digestive drugs, hormones, urogenital drugs, anal drugs, dermatological drugs, dental and oral cavity agents, vitamins, revitalizers, hematological agents, humoral agents, agents for artificial dialysis, liver disease drugs, antidotes, agents for habitual intoxication, gout remedies, enzymes, diabetes drugs, cell activators, anti-tumor agents, radioactive drugs, antiallergic agents, herbal medicines, antibiotics, drugs for chemotherapy, vaccines, agents against parasites, diagnostic agents, narcotics, stimulant drugs and the like.

More specific examples of the water-soluble active substance to be contained in the seamless capsule of the present invention comprise one or more active substance (s) and the like selected from the group consisting of acetylspiramycin, amoxicillin, ethyl icosapentate, itraconazole, oxatomide, olopatadine, glybuzole, glutathione, ketophenylbutazone, cobamamide, cisapride, todralazine, tropisetron, domperidone, valproic acid, pyridoxal, fluorouracil, flunarizine, flurazepam, benidipine, minocycline, mebendazole, medroxyprogesterone, ubidecarenone, levodopa, salts thereof and the like.

Here, examples of the salts include acid addition salts such as inorganic acid salts including a hydrochloride, a hydrobromide, a nitrate, a sulfate and a phosphate and the like, and organic acid salts including a benzenesulfonate, a benzoate, a citrate, a fumarate, a gluconate, a lactate, a maleate, a maliate, an oxaliate, a methanesulfonate, a tartarate and the like, metal salts such as alkali metal salts including a sodium salt, a potassium salt and the like, alkaline earth metal salts including a magnesium salt, a calcium salt and the like, an aluminum salt and a zinc salt, ammonium salts such as ammonium and tetramethylammonium, organic amine addition salts such as salts with morpholine and piperidine, amino acid addition salts such as salts with glycine, phenylalanine, lysine, aspartic acid and glutamic acid and the like.

The weight ratio of the water-soluble active substance to the oily compound to be comprised in the content of the seamless capsule of the present invention is not particularly limited, however, it is preferably 20:80 to 0.01:99.99, more preferably 5:95 to 0.1:99.9. Further, in order for the water-soluble active substance to be easily suspended in the oily compound, the particle size of the water-soluble active substance is preferably 50 µm or less, more preferably 20 µm or less in terms of a volume average particle size measured by microscopy or a sieving method.

The shell of the seamless capsule of the present invention may be any shell as long as it is conventionally used a seamless capsule. For example, a shell comprising an encapsulating compound such as gelatin, agar, sodium alginate, carrageenan or pectin is preferred, and a shell comprising gelatin or agar is more preferred. For example, in the case of a shell that comprises agar, it is preferred that the agar can be dissolved in water, for example, at 80 to 100°C, and it is preferred that it can be dissolved in at least boiling water.

A encapsulating solution to be used in the process for producing the seamless capsule of the present invention is, for example, an aqueous solution or the like containing a encapsulating compound such as gelatin, agar, sodium alginate, carrageenan or pectin. The concentration of the encapsulating compound in the aqueous solution is preferably, for example, not higher than a concentration at which the encapsulating compound can be dissolved in boiling water, and not lower than a minimum concentration at which a shell is formed at the temperature of a cooling medium. For example, in the case where a shell that comprises agar is prepared, the concentration of agar in an aqueous solution of agar is preferably 0.5 to 10%, more preferably 1 to 5%. The ratio of the above-mentioned encapsulating compound to the shell is preferably 5 to 100 parts by weight relative to 100 parts by weight of the shell.

It is possible to comprise a plasticizer in the shell of the seamless capsule of the present invention. Examples of the plasticizer include glycerol, sugars, sugar alcohols, water-soluble polymers and the like, and preferred examples include sugars and sugar alcohols having less than 24 carbon atoms and the like. Specific examples thereof include sorbitol, sucrose, lactose, maltose, erythritol, trehalose, xylitol, lactitol, mannitol, and the like. The ratio of the plasticizer to the shell is preferably 0 to 95 parts by weight relative to 100 parts by weight of the shell.

In the shell of the seamless capsule of the present invention, a sweetener, a perfume, a coloring agent, an antistatic agent, a flavor, a smell-corrective, a stabilizer, a disintegrator or the like can be comprised. As the stabilizer, a light-blocking agent and the like can be exemplified. Examples of the light-blocking agent include titanium oxide, iron oxide, magnesium oxide, calcium oxide, zinc oxide, calcium carbonate, talc, silicon oxide, an aluminosilicate, sodium copper chlorophyllin and an aluminum lake of tar dye and the like. Further, it is preferred that as the disintegrator, one or more substance(s) selected from, for example, pullulan, hydroxypropyl starch, solubilized starch, dextrin, sodium carboxymethyl starch, acasia, alginic acid, sodium alginate, propylene glycol alginate, carrageenan and the like are comprised. The ratio of such a sweetener, perfume, coloring agent, antistatic, agent, flavor, smell-corrective, stabilizer, disintegrator or the like to the shell is preferably 0 to 40 parts by weight in total relative to 100 parts by weight of the shell.

The seamless capsule of the present invention comprises an intermediate layer laying between the content and the shell. The intermediate layer comprises an oily compound which does not dissolve or merge with above-mentioned the water-soluble active substance, is not dissolved in or merged with water, has a melting point of 10 to 65°C, and is not dissolved in or merged with the content at a temperature not higher than the melting point during the production process of the seamless capsule. Incidentally, as the oily compound constituting the content, a water-soluble oily compound can also be used, however, it is preferred that an oily compound which dose not penetrate water is used as the oily compound constituting the intermediate layer. Incidentally, it is necessary that the oily compound constituting the intermediate layer does not dissolve or merge with the water-soluble active substance and is not dissolved in or merged with the content at a temperature not higher than the melting point during the production process of the seamless capsule. However, it does not matter that the oily compound is slowly dissolved in or merged with the content in the case where it is placed at a temperature not lower than the melting point in the production process of the seamless capsule or after the production.

Further, the oily compound constituting the intermediate layer of the seamless capsule of the present invention is preferably an oily compound having a melting point of not higher than 37°C, which is a healthy human body temperature. When the oily compound constituting the intermediate layer has a melting point of not higher than 37°C, it becomes easy for the water-soluble active substance to be promptly released from the seamless capsule in the gastrointestinal tract after oral administration. On the other hand, for example, it is also possible to use an oily compound having a melting point of 37°C or higher as the oily compound constituting the intermediate layer of the seamless capsule in the case where the water-soluble active substance is to be released by breaking down the seamless capsule with a digestive enzyme, an enteric bacterium or the like, or in the case where the seamless capsule is an external preparation and the water-soluble active substance is to be released by breaking down the seamless capsule upon applying the preparation. Further, even if the melting point of the oily compound constituting the intermediate layer is 37°C or higher, it is also possible for the water-soluble active substance to be promptly released from the capsule in the gastrointestinal tract after oral administration by setting the melting point of a mixture obtained by dissolving or mixing the oily compound constituting the intermediate layer in the oily compound constituting the content when the seamless capsule is stored at a temperature not lower than room temperature, preferably at a temperature not lower than the melting point of the oily compound constituting the intermediate layer after the production of the seamless capsule to 37°C or lower.

Further, at least in the production of the seamless capsule of the present invention, it is necessary to set the temperature during the production to a temperature not higher than the melting point of the oily compound constituting the intermediate layer so that the oily compound constituting the intermediate layer of the seamless capsule does not dissolve or merge with the water-soluble active substance and is not dissolved in or merged with the content. However, the temperature during drying is preferably 25°C or higher from the viewpoint of the drying efficiency or energy consumption. In the light of this, the oily compound(s) constituting the intermediate layer is preferably an oily compound having a melting point of not lower than 25°C, which is a standard room temperature.

As the oily compound constituting the intermediate layer of the seamless capsule of the present invention, for example one or more oily compound(s) selected from hard fats, cacao oil, lard, hydrogenated oil, partially hydrogenated oil, glycerol esters of fatty acids, fatty acid acyl esters and paraffin are preferred, and among these, those with a melting point of 10 to 65°C, preferably 25 to 37°C are preferred. Further, a mixture obtained by mixing one or more high-melting oil selected from hard fats, cacao oil, lard, hydrogenated oil, partially hydrogenated oil, high-melting glycerol esters of fatty acids, high-melting fatty acid acyl esters, paraffin and the like with one or more low-melting oil selected from plant oil, low-melting glycerol esters of fatty acids, low-melting fatty acyl esters, liquid paraffin and the like such that the resulting mixture has a melting point of 10 to 65°C, preferably 25 to 37°C can also be used. As the oily compound constituting the intermediate layer, more preferably a mixture obtained by mixing one or more substance(s) selected from hard fats and cacao oil or one or more high-melting oil selected from hard fats, cacao oil and saturated long-chain fatty acid triglycerides with one or more low-melting oil selected from soybean oil, castor oil, canola oil and unsaturated long-chain fatty acid triglycerides such that the resulting mixture has a melting point of 10 to 65°C, more preferably 25 to 37°C and the like can be exemplified. Incidentally, when a mixture of high-melting oil and low-melting oil is used, the ratio of the high-melting oil to the low-melting oil is preferably 1:4 to 4:1, and in this occasion, it is preferred that oil with similar structures to each other is used.

The seamless capsule of the present invention can be produced by, for example, a conventional process for producing a mono-nuclear multi-layered seamless capsule. Specifically, for example, it can be produced by a production process comprising the steps of: with the use of a device for producing a seamless capsule equipped with three or more layered nozzles with sequentially increasing diameters, forming a multi-layered droplet by continuously discharging to a cooling medium, the above-mentioned encapsulating solution from the outermost nozzle, an oily compound constituting an intermediate layer comprising the oily compound which does not dissolve or merge with a water-soluble active substance, is not dissolved in or merged with water, has a melting point of 10 to 65°C, and is not dissolved in or merged with content of the seamless capsule at a temperature not higher than the melting point during the production process of the seamless capsule from at least one nozzle other than the innermost nozzle among nozzles other than the outermost nozzle, and the above-mentioned content comprising the water-soluble active substance and an oily compound from at least one nozzle inside the nozzle discharging the oily compound constituting the intermediate layer, respectively; hardening or gelating the oily compound constituting the intermediate layer by cooling the multi-layered droplet to a temperature not higher than the melting point of the oily compound constituting the intermediate layer at the same time as or immediately after the formation of the droplet; and drying the resulting solid matter at a temperature not higher than the melting point of the oily compound constituting the intermediate layer.

Examples of the production device to be used in the production of the seamless capsule of the present invention include a seamless minicapsule production device (SPHEREX, manufactured by Freund Industrial Co. Ltd.) and the like.

The cooling medium to be used in the production of the seamless capsule of the present invention may be any liquid as long as, for example, it is not promptly merged with water and does not promptly dissolve a substance such as agar or gelatin comprised in the shell. Further, it may be cooled distilled water or a cooled buffer as long as its temperature is sufficiently low. More preferably, oil which form an interface with water and in which the shell is slightly soluble and the like can be exemplified. Examples thereof include one type of liquid or a liquid mixture of two or more types of liquids selected from oil and fats such as medium-chain fatty acid triglycerides and soybean oil, polar organic solvents such as ethyl acetate, isooctanol, cyclohexanone and n-amylalcohol, non-polar hydrocarbons such as liquid paraffin and normal paraffin having 6 or more carbon atoms, and the like.

In the above-mentioned production of the seamless capsule of the present invention, the above-mentioned encapsulating solution can be prepared by, for example, adding a encapsulating compound such as gelatin, agar, sodium alginate, carrageenan or pectin and the like to water and heating the mixture until it becomes a flowable liquid by dissolving or dispersing the encapsulating compound or the like therein. The encapsulating solution is used in the production of the seamless capsule while maintaining the temperature at a temperature that does not allow the encapsulating compound and the like to be deposited from the encapsulating solution or does not allow the encapsulating solution to be solidified. Also, the oily compound constituting the intermediate layer is heated until the substance becomes a flowable liquid and is used in the production of the seamless capsule while maintaining the temperature at a temperature that does not allow the liquid to be solidified. Also, it is necessary to keep the above-mentioned encapsulating solution and the oily compound constituting the intermediate layer be kept warm so that they are in a state of flowable liquid until they reach the nozzles of the above-mentioned production device, respectively, during the production of the seamless capsule.

In the production of the seamless capsule of the present invention, it is preferred that the intermediate layer is cooled to a temperature not higher than the melting point of the oily compound constituting the intermediate layer at the same time as or immediately after the formation of the droplet. Because by doing this, the intermediate layer is hardened or gelated, the water-soluble active substance does not pass through the solidified or semi-solidified intermediate layer, and is protected from coming into contact with the shell that contains water, whereby the water-soluble active substance can be protected from becoming unstable. In the case where the oily compound constituting the intermediate layer is dissolved in or merged with the content at a temperature not lower than the melting point of the oily compound, it is preferred that the intermediate layer is cooled to a temperature not higher than the melting point of the oily compound at least before all the oily compound constituting the intermediate layer is dissolved in or merged with the content.

In the above-mentioned production of the seamless capsule of the present invention, in order to harden or gelate the oily compound constituting the intermediate layer at the same time as or immediately after the formation of the droplet, it is preferred that the cooling medium is cooled in advance to a temperature not higher than the melting point of the oily compound constituting the intermediate layer, more preferably not higher than 20°C, further more preferably not higher than 15°C.

The difference in temperature between the above-mentioned encapsulating solution and the oily compound constituting the intermediate layer, preferably, the difference in temperature among the above-mentioned encapsulating solution, the oily compound constituting the intermediate layer and the content is preferably within 30°C, more preferably within 15°C.

It is preferred that the content comprising the water-soluble active substance and the oily compound, the oily compound constituting the intermediate layer and the encapsulating solution are discharged from nozzles at a speed ratio of 1:0.1:1 to 1:10:30, more preferably 1:0.5:1 to 1:3:10.

In the production process of the seamless capsule of the present invention, because right after the oily compound constituting the intermediate layer is hardened or gelated by cooling the multi-layered droplet, the resulting solid matter contains a great amount of water, it is necessary to dry it by using, for example, a rotary drum dryer or the like. Also in the drying step, the water-soluble active substance can be moved to the intermediate layer and the shell by the action of the rotational motion or the gravity, it is necessary to maintain the intermediate layer in a solid or semi-solid state. Therefore, it is preferred that the drying step is carried out at a temperature not higher than the melting point of the oily compound constituting the intermediate layer. However, it does not matter that in order to fully dry out the shell, the temperature is increased to a temperature not lower than the melting point of the oily compound constituting the intermediate layer after the water contained in the shell is almost removed.

On the other hand, as preliminary drying, after the oily compound constituting the intermediate layer is hardened or gelated by cooling the multi-layered droplet, water may be removed in advance by immersing the resulting solid matter in ethanol or a liquid containing ethanol. This procedure may be carried out by the method described in Japanese Patent No. 2784872 or JP-A-10-211425. For example, in the case where the shell of the seamless capsule comprises a encapsulating compound and one or more substance(s) selected from sugars, sugar alcohols and hydrophilic polymers that are very slightly soluble in ethanol, very few substances are removed from the seamless capsule by ethanol, leading to obtaining the seamless capsule without degrading the performance, therefore, this method is useful.

The seamless capsule of the present invention can also be sieved with a sieve or an equivalent one. On this occasion, by adjusting the openings of the sieve, it is possible to obtain pills, granules, fine granules and powdered drugs described in the Japanese pharmacopoeia, respectively. Further, the seamless capsule may be filled in a hard capsule, bottle or the like, or may be packed as a divided preparation. Further, before filling or packing the seamless capsule, an antistatic agent, a flavor, a smell-corrective or the like may be added in a hard capsule, bottle or the like.

Further, the seamless capsule of the present invention may be compression molded with a tableting machine, thereby to prepare a compressed preparation. Examples of the compressed preparation include oral tablets such as tablets for oral administration, foaming tablets, enteric tablets, chewable tablets, intraorally rapidly disintegrable tablets, troches and buccal or sublingual tablets and the like. Further, the compressed preparation may be a multiple-unit tablet or a functional tablet such as a gradumet, a wax matrix, a resinate or a spantab.

In the case where the seamless capsule of the present invention is compression molded with a tableting machine, the seamless capsule is mixed with various additives, and if desired, a lubricant is mixed therewith to prepare a tableting powder, and tableting can be carried out with a known tableting machine, preferably a rotary tableting machine or a single-shot tableting machine, which achieves high productivity. Further, at the time of compression molding, it is also possible to perform compression molding by applying a lubricant on a punch and a die of a tableting machine in advance without mixing the lubricant with the above-mentioned powder mixture.

Hereinafter, the present invention will be described in more detail with reference to Examples, however, the invention is not limited to these Examples.

### Example 1

As content, 20 g of olopatadine hydrochloride was added to 3980 g of soybean oil and well suspended therein. As an oily compound constituting an intermediate layer, 1000 g of a hydrogenated oil (shortening PW-50, YOKOZEKI OIL & FAT IND. CO. , LTD.) was heated to 70°C or higher and melted. In addition, as an encapsulating solution, to 60 g of agar and 700 g of sucrose, 1240 g of purified water was added and they were well mixed and heated to 80 to 100°C in a water bath and dissolved.

With the use of a seamless minicapsule production device (SPHEREX, manufactured by Freund Industrial Co. Ltd.), the content, the oily compound constituting the intermediate layer and the encapsulating solution were continuously discharged at rates of 5.5 mL, 4.9 mL and 21.4 mL per minute, respectively, using concentric three-layered nozzles to a medium-chain fatty acid triglyceride (Coconad MT, Kao Corporation) cooled to about 15°C, whereby a mono-nuclear two-layered seamless capsule having a diameter of about 1.5 mm was produced. Then, the resulting seamless capsule was separated from the medium-chain fatty acid triglyceride and dried at 25°C and a relative humidity of 30% using a rotary dryer.

### Example 2

As content, 20 g of olopatadine hydrochloride was added to 3980 g of soybean oil and well suspended therein. As an oily compound constituting an intermediate layer, 500 g of a glycerol fatty acid ester (Witepsol H-15, Mitsuba Trading Co. , Ltd.) was added to 500 g of soybean oil, and the mixture was heated to 50°C or higher and melted. In addition, as a encapsulating solution, to 60 g of agar and 700 g of sucrose, 1240 g of purified water was added and they were well mixed and heated to 80 to 100°C in a water bath and dissolved.

With the use of a seamless minicapsule production device (SPHEREX, manufactured by Freund Industrial Co. Ltd.), the content, the oily compound constituting the intermediate layer and the encapsulating solution were continuously-discharged at rates of 5.5 mL, 4.9 mL and 21.4 mL per minute, respectively, using concentric three-layered nozzles to a medium-chain fatty acid triglyceride (Coconad MT , Kao Corporation) cooled to about 15°C, whereby a mono-nuclear two-layered seamless capsule having a diameter of about 1.5 mm was produced. Then, the resulting seamless capsule was separated from the medium-chain fatty acid triglyceride and dried at 25°C and a relative humidity of 30% using a rotary dryer.

### Example 3

As content, 40 g of olopatadine hydrochloride was added to 3960 g of a medium-chain fatty acid triglyceride (Coconad MT, Kao Corporation) and well suspended therein. As an oily compound constituting an intermediate layer, 2000 g of cocoa oil was heated to 50°C or higher and melted. In addition, as a encapsulating solution, to 60 g of agar and 700 g of sucrose, 1240 g of purified water was added and they were well mixed and heated to 80 to 100°C in a water bath and dissolved.

With the use of a seamless minicapsule production device (SPHEREX, manufactured by Freund Industrial Co. Ltd.), the content, the oily compound constituting the intermediate layer and the encapsulating solution were continuously discharged at rates of 5.5 mL, 6.0 mL and 19. 7 mL per minute, respectively, using concentric three-layered nozzles to a medium-chain fatty acid triglyceride (Coconad MT, Kao Corporation) cooled to about 15°C, whereby a mono-nuclear two-layered seamless capsule having a diameter of about 1.5 mm was produced. Then, the resulting seamless capsule was separated from the medium-chain fatty acid triglyceride and dried at 25°C and a relative humidity of 30% using a rotary dryer.

### Example 4

As content, 40 g of olopatadine hydrochloride was added to 3960 g of a medium-chain fatty acid triglyceride (Coconad MT, Kao Corporation) and well suspended therein. As an oily compound constituting an intermediate layer, 2000 g of cocoa oil was heated to 50°C or higher and melted. In addition, as a encapsulating solution, to 50 g of agar, 590 g of sucrose and 160 g of titanium oxide, 1200 g of purified water was added and they were well mixed and heated to 80 to 100°C in a water bath and dissolved.

With the use of a seamless minicapsule production device (SPHEREX, manufactured by Freund Industrial Co. Ltd.), the content, the oily compound constituting the intermediate layer and the encapsulating solution were continuously discharged at rates of 5.5 mL, 9.5 mL and 17.0 mL per minute, respectively, using concentric three-layered nozzles to a medium-chain fatty acid triglyceride (Coconad MT, Kao Corporation) cooled to about 15°C, whereby a mono-nuclear two-layered seamless capsule having a diameter of about 1.5 mm was produced. Then, the resulting seamless capsule was separated from the medium-chain fatty acid triglyceride and dried at 25°C and a relative humidity of 30% using a rotary dryer.

### Comparative Example 1

As content, 20 g of olopatadine hydrochloride was added to 3980 g of soybean oil and well suspended therein. As a encapsulating solution, to 60 g of agar and 700 g of sucrose, 1240 g of purified water was added and they were well mixed and heated to 80 to 100°C in a water bath to dissolve the agar.

With the use of a seamless minicapsule production device (SPHEREX, manufactured by Freund Industrial Co. Ltd.), the content and the encapsulating solution were continuously discharged at rates of 5.5 mL and 21.4 mL per minute, respectively, using concentric two-layered nozzles to a medium-chain fatty acid triglyceride (Coconad MT, Kao Corporation) cooled to about 15°C, whereby a mono-nuclear single-layered seamless capsule having a diameter of about 1.5 mm was produced. Then, the resulting seamless capsule was separated from the medium-chain fatty acid triglyceride and dried at 25°C and a relative humidity of 30% using a rotary dryer.

### Comparative Example 2

As content, 40 g of olopatadine hydrochloride was added to 3960 g of a medium-chain fatty acid triglyceride (Coconad MT, Kao Corporation) and well suspended therein. As a encapsulating solution, to 60 g of agar and 700 g of sucrose, 1240 g of purified water was added and they were well mixed and heated to 80 to 100°C to dissolve the agar.

With the use of a seamless minicapsule production device (SPHEREX, manufactured by Freund Industrial Co. Ltd.), the content and the encapsulating solution were continuously discharged at rates of 6.2 mL and 9.9 mL per minute, respectively, using concentric two-layered nozzles to a medium-chain fatty acid triglyceride (Coconad MT, Kao Corporation) cooled to about 15°C, whereby a mono-nuclear single-layered seamless capsule having a diameter of about 1. 5 mm was produced. Then, the resulting seamless capsule was separated from the medium-chain fatty acid triglyceride and dried at 25°C and a relative humidity of 30% using a rotary dryer.

### Test example 1

The respective seamless capsules obtained in Examples 1 to 4 and Comparative Examples 1 and 2 were exposed to 1,000 Lux of light of D65 lamp at 15°C and a relative humidity of 60% for 50 days. Then, the production amount of a geometric isomer (Compound A) of olopatadine hydrochloride was measured by high performance liquid chromatography, and the production ratio thereof to the olopatadine hydrochloride at the initiation of light exposure was determined. The conditions for high performance liquid chromatography are as follows.
Column: Inertsil C8 4.6 mm x 250 mm (GL Science Inc.)
Column temperature: Constant temperature at around 40°C
Mobile phase: [0.05 mol/L phosphate buffer (pH 3.5) :
acetonitrile = 550 mL : 450 mL] + 2.3 g of sodium lauryl sulfate Detection method: UV absorptiometry (wavelength of 299 nm)

As a result, as shown in Table 1, while the production ratios of Compound A were high in the seamless capsules obtained in Comparative Examples 1 and 2, the production ratios of Compound A were low in the seamless capsules obtained in Examples 1 to 4.

From these results, it can be said that in the seamless capsule of the present invention, the water-soluble active substance comprised in the content was protected from becoming unstable.

**[Table 1]**

| Production ratio of photoreaction product A in photostability test of seamless capsule (25°C, 75% RH, D65 lamp, 1,200,000 lx·h) | |
|---|---|
| Seamless capsule | Production ratio of Compound A (%) |
| Example 1: soybean oil/ hydrogenated oil/ agar | 2.8 |
| Example 2: soybean oil/ glycerol fatty acid ester·soybean oil/ agar | 3.9 |
| Comparative Example 1: soybean oil/ agar | 19.5 |
| Example 3: medium-chain fatty acid triglyceride/ cacao oil/ agar | 3.0 |
| Example 4: medium-chain fatty acid triglyceride/ cacao oil/ agar·titanium oxide | 1.2 |
| Comparative Example 2: medium-chain fatty acid triglyceride/ agar | 14.9 |

### Test example 2

For the seamless capsules obtained in Examples 2 to 4, an elution test described in the Japanese Pharmacopoeia 14th edition was carried out using water as a test liquid. As a result, in any of the seamless capsules, 85% or more of the comprised olopatadine hydrochloride was eluted in 30 minutes.

That is, it can be said that the seamless capsule of the present invention is a seamless capsule from which the water-soluble active substance is promptly released in the gastrointestinal tract after oral administration.

### Industrial Applicability

According to the present invention, a seamless capsule containing a water-soluble active substance in which the water-soluble active substance is protected from becoming unstable, and a process for producing the same can be provided.

## Claims

1. A seamless capsule comprising: content comprising a water-soluble active substance and an oily compound; a shell covering the content; and an intermediate layer, which is laying between the content and the shell and comprises an oily compound which does not dissolve or merge with the water-soluble active substance, is not dissolved in or merged with water, has a melting point of 10 to 65°C, and is not dissolved in or merged with the content at a temperature not higher than the melting point during the production process of the seamless capsule.

2. The seamless capsule according to claim 1, wherein the intermediate layer is an intermediate layer comprising an oily compound having a melting point of 25 to 37°C.

3. The seamless capsule according to claim 1 or 2, wherein the intermediate layer is an intermediate layer comprising one or more oil selected from hard fats, cacao oil, lard, hydrogenated oil, partially hydrogenated oil, glycerol esters of fatty acids, fatty acid acyl esters and paraffin.

4. The seamless capsule according to claim 1 or 2, wherein the intermediate layer is an intermediate layer comprising a mixture of one or more oil selected from hard fats , cacao oil, lard, hydrogenated oil, partially hydrogenated oil, high-melting glycerol esters of fatty acids, high-melting fatty acid acyl esters and paraffin with one or more oil selected from plant oil, low-melting glycerol esters of fatty acids, low-melting fatty acid acyl esters and liquid paraffin.

5. The seamless capsule according to any one of claims 1 to 4, wherein the shell is a shell comprising gelatin or agar.

6. The seamless capsule according to claim 5, wherein the shell is a shell comprising one or more substance(s) selected from titanium oxide, iron oxide, magnesium oxide, calcium oxide, zinc oxide, calcium carbonate, talc, silicon oxide, aluminosilicates, sodium copper chlorophyllin and aluminum lakes of tar dyes.

7. The seamless capsule according to any one of claims 1 to 6, wherein the water-soluble active substance is olopatadine or a salt thereof.

8. A process for producing a seamless capsule comprising a water-soluble active substance, which comprises the steps of: with the use of a device for producing the seamless capsule equipped with three or more layered nozzles with sequentially increasing diameters, forming a multi-layered droplet by continuously discharging to a cooling medium, a encapsulating solution from the outermost nozzle, an oily compound constituting an intermediate layer comprising the oily compound which does not dissolve or merge with a water-soluble active substance, is not dissolved in or merged with water, has a melting point of 10 to 65°C, and is not dissolved in or merged with content of the seamless capsule at a temperature not higher than the melting point during the production process of the seamless capsule from at least one nozzle other than the innermost nozzle among nozzles other than the outermost nozzle, and the content comprising the water-soluble active substance and an oily compound from at least one nozzle inside the nozzle discharging the oily compound constituting the intermediate layer, respectively; hardening or gelating the oily compound constituting the intermediate layer by cooling the multi-layered droplet to a temperature not higher than the melting point of the oily compound constituting the intermediate layer at the same time as or immediately after the formation of the droplet; and drying the resulting solid matter at a temperature not higher than the melting point of the oily compound constituting the intermediate layer.

9. A seamless capsule containing a water-soluble active substance that can be produced by the process for producing a seamless capsule according to claim 8.
